# NEW EUROPEAN PATENT SPECIFICATION

(11) **EP 1 194 580 B2**
(45) Date of publication and mention of the opposition decision: **25.08.2010**
(45) Mention of the grant of the patent: 07.02.2007
(21) Application number: 00946097.3
(22) Date of filing: 14.07.2000
(51) Int. Cl.: C12N 15/86, C12N 7/01, C12N 5/10, A61K 35/76, A61K 39/145

(54) **IN VITRO RECONSTITUTION OF SEGMENTED NEGATIVE-STRAND RNA VIRUSES**
IN VITRO-REKONSTITUTION VON SEGMENTIERTEN, NEGATIVSTRANG-RNA-VIREN
RECONSTITUTION IN VITRO DE VIRUS A ARN DE POLARITE NEGATIVE SEGMENTES

(30) Priority: 14.07.1999 US 143645 P; 16.07.1999 GB 9916794
(43) Date of publication of application: 10.04.2002
(73) Proprietor: Mount Sinai School of Medicine of New York University, New York, NY 10029 (US)
(72) Inventor: BROWNLEE, George, Gow, University of Oxford, Oxford OX1 3RE (GB); FODOR, Ervin, University of Oxford, Oxford OX1 3RE (GB); PALESE, Peter, Mount Sinai School of Med. NY Uni., New York, NY 10029 (US); GARCIA-SASTRE, Adolfo, M. Sinai Sch. Med. NY Uni., New York, NY 10029 (US)
(74) Representative: Weber, Martin
(86) International application number: PCT/GB2000/002710
(87) International publication number: WO 2001/004333

(56) References cited:
- WO-A-00/60050
- WO-A-96/10632
- PLESCHKA S. ET AL.: "A Plasmid-Based Reverse Genetics System for Influenza A Virus" JOURNAL OF VIROLOGY, vol. 70, no. 6, June 1996 (1996-06), pages 4188-4192, XP002150092 ISSN: 0022-538X cited in the application
- MENA I. ET AL.: "Rescue of a Synthetic Chloramphenicol Acetyltransferase RNA into Influenza Virus-Like Particles Obtained from Recombinant Plasmids" JOURNAL OF VIROLOGY, vol. 70, no. 8, August 1996 (1996-08), pages 5016-5024, XP002150091 ISSN: 0022-538X
- FODOR E. ET AL.: "Rescue of influenza A virus from recombinant DNA." JOURNAL OF VIROLOGY, vol. 73, no. 11, November 1999 (1999-11), pages 9679-9682, XP002151487
- NEUMANN G. ET AL.: "Generation of influenza A viruses entirely from cloned cDNAs." PROCEEDINGS OF THE NATIONAL ACADEMY OF SCIENCES OF THE UNITED STATES, vol. 96, no. 16, 3 August 1999 (1999-08-03), pages 9345-9350, XP002151639 Aug. 3, 1999 ISSN: 0027-8424

## Description

The present invention relates to reconstitution (rescue) of influenza A virus in cultured cells from recombinant DNAs. More particularly, it relates to rescue of influenza A virus having generally 8 genomic vRNA segments, by an entirely vector-driven system, which avoids the need for a helper virus.

The past five years have witnessed the rescue of most of the important non-segmented negative-strand RNA viruses from recombinant DNA. First, Schnell et al. succeeded in the recovery of rabies virus from recombinant DNA (EMBO J. (1994) 13, 4195-4203). Shortly after, plasmid-based rescue systems were developed for vesicular stomatitis virus (Lawson et al., Proc. Natl. Acad. Sci. USA (1995) 92, 4477-4481;Whelan et al., Proc. Natl. Acad. Sci. USA (1995) 92, 8388-8392), respiratory syncytical virus (Collins et al., Proc. Natl. Acad. Sci. USA (1995) 92, 11563-11567;Jin et al., Virology (1998)251, 206-214), measles virus (Radecke et al., EMBO J. (1995) 14, 5773-5784) and sendai virus (Garcin et al., EMBO J. (1995) 14, 6087-6094; Kato et al., Genes Cells (1996) 1, 569-579). More recently, plasmid-based rescue systems have been developed for human parainfluenza type 3 (Durbin et al., Virology (1997) 235, 323-332; Hoffman and Banerjee, J. Virol.(1997) 71, 4272-4277), rinderpest virus (Baron and Barrett, J. Virol. (1997) 71, 1265-1271), simian virus 5 (He et al., Virology (1997) 237, 249-260), bovine respiratory syncytical virus (Buchholz et al., J. Virol. (1999) 73, 251-259) and Newcastle disease virus (Peeters et al., J. Virol. (1999) 73, 5001-5009).

Bridgen and Elliott (Proc. Natl. Acad. Sci. USA (1996) 14, 15400-15404) have reported helper virus-free rescue of a bunyavirus having just 3 genomic vRNA segments from cDNA by expressing anti-genome RNAs and viral proteins under the control of a bacteriophage T7 promoter. However, up to now it has not been known whether a totally plasmid-based strategy can be successful applied for rescuing segmented negative-strand RNA viruses with a greater number of vRNA segments, such as influenza viruses, without the aid of a helper virus.

Influenza remains a constant worldwide threat to human health and hence there is a particular need for a ready method of generating modified influenza viruses with known mutations in any of the genomic vRNA segments. Engineering of influenza vRNA segments for expression of heterologous sequences is also of much interest, for example, in the development of new vaccines effective against influenza virus and a second pathogenic agent. Three types of influenza virus are known designated as types A, B and C. Up to now, influenza A has been the main focus of attention as regards genetic manipulation. The genome of a wild-type influenza A virus consists of 8 segments of single-stranded negative sense RNA which encode 10 polypeptides: the RNA-dependent RNA polymerase proteins (PB 1, PB2, and PA), the nucleoprotein (NP), the matrix proteins (M1, M2), two surface glycoproteins which project from the lipoprotein envelope (haemagglutinin (HA) and neuraminidase (NA)) and the non-structural proteins NS1 and NS2. The HA, NA, NP and polymerase proteins are encoded by monocistronic genomic segments.

As for other segmented negative-strand RNA viruses, during the replication cycle of an influenza virus the viral genome is transcribed into mRNA and replicated into complementary RNA (cRNA). For this the genomic vRNA segments need to be complexed with the nucleoprotein and RNA-dependent RNA polymerase in ribonucleoprotein (RNP) complexes (Huang et al., J. Virol. (1990) 64, 5669-5673; García-Sastre, Trends In Biotechnology (1998) 16, 230-235). Initially, in order to manipulate the genome of an influenza virus, RNPs were reconstituted *in vitro* from RNA transcribed from plasmid DNA in the presence of the polymerase proteins PB1, PB2 and PA and the nucleoprotein isolated from purified influenza virus (Enami et al., Proc. Natl. Acad. Sci. USA (1990) 87, 3802-3805; Enami and Palese, J. Virol. (1991) 65, 2711-2713; Muster and Garcia-Sastre, Genetic manipulation of influenza viruses in Textbook of influenza (1998), ch. 9, eds. Nicholson et al.). The *in vitro* reconstituted RNPs were transfected into cells infected with a helper influenza virus, which provided the remaining required viral proteins and RNA segments, resulting in generation of transfectant viruses. This technique has been extremely useful in advancing understanding of the molecular biology and pathogenicity of influenza viruses. However, it relies on highly specialised selection methods to isolate the transfectant viruses from the helper virus, which restricts its use to certain RNA segments of a limited number of viral strains.

More recently, intracellular reconstitution of an RNP complex from an intracellularly transcribed vRNA segment has been shown to be possible using plasmid-expressed NP and polymerase proteins (Neumann et al., Virology (1994) 202, 477-479; Zhang and Air, Biochem. Biophys. Res. Commun. (1994) 200, 95-101; Pleschka et al., J. Virol. (1996) 70, 4188-4192). For example, Pleschka et al. showed that influenza A PB 1, PB2, PA and NP expressed from plasmids could encapsidate, transcribe and replicate an influenza virus vRNA-like RNA containing a chloramphenicol acetyltransferase (CAT) reporter gene in transfected human 293 cells. This vRNA-like reporter gene was introduced into the chosen host cells by transfection of a plasmid DNA (pPOLI-CAT-RT) having a truncated human RNA polymerase I promoter (nucleotides -250 to -1) positioned upstream of the vRNA-coding region. The sequence of the hepatitis delta virus genomic ribozyme was positioned downstream of the vRNA-coding region in order to ensure RNA processing to give the correct 3' end of the transcribed vRNA. It was also reported by the same group that by replacing the plasmid encoding the CAT reporter gene with a plasmid encoding an authentic influenza A vRNA segment, intracellularly reconstituted RNP complexes could be rescued into transfectant viruses upon infection of the transfected cells with an influenza helper virus. Such a helper virus-based rescue system was investigated by Pleschka et al. employing intracellularly transcribed mutant influenza A NA vRNA segments. Moving to an entirely plasmid-based rescue system for influenza viruses raises, however, new considerations in terms of obtaining adequate expression of all the required sequences and the correct cooperation of those sequences to assemble a complete virus.

Bridgen and Elliott were unable to rescue Bunyamwera bunyvirus using plasmids directing expression of genomic vRNA segments rather than anti-genome RNAs. It has now been found, however, that helper virus-free rescue of an influenza A virus in cell culture is feasible by cotransfection into cultured cells of 12 plasmids capable of corexpressing 8 vRNA segments and the required NP and 3 RNA polymerase proteins for formation of RNP complexes. This same strategy can be extrapolated to a variety of other segmented negative-strand RNA viruses having a large number of genomic vRNA segments, for example 6 or more, including, for example, influenza viruses of other types and other members of the family Orthomyxoviridae such as the 6 vRNA segment-containing thogotoviruses. While both wild-type influenza A and influenza B viruses have 8 vRNA segments, modified influenza A viruses have been described containing an additional vRNA segment (Enami et al., Virology (1993) 185, 765-90) and influenza C viruses have one less vRNA segment.

### Summary of the invention

The present invention thus provides a method for generating infectious viral particles of an influenza A virus, said method comprising:
introducing into cultured cells expression vectors that express in said cells the complete genomic vRNA segments of said virus, wherein said cells support growth of said virus, said cells also providing a nucleoprotein and RNA-dependent RNA polymerase whereby RNP complexes containing the genomic vRNA segments of said virus are formed and said infectious viral particles are produced by said cells in the absence of a helper virus and wherein said cells do not produce interferon.

The chosen host cells may, for example, additionally have introduced one or more further expression vectors capable of directing expression in the cells of one or more of the required viral proteins. For example, a second set of expression vectors may be employed to express in the host cells all of the nucleoprotein and RNA-dependent polymerase subunits. Conveniently, for example, a separate expression vector for each of those proteins may be employed. The chosen host cells may, for example, alternatively be engineered to express one or more of the required nucleoprotein and RNA-dependent RNA polymerase subunits. If all the essential proteins for encapsidation, transcription and replication of the vRNAs are provided by the starting host cells, then it will be appreciated that only the first set of expression vectors is required. Furthermore, the chosen starting host cells may be cells engineered to express one or more of the genomic vRNAs segments of the desired virus. By means of a method of the invention, it will be appreciated that rescue of an influenza A virus can be achieved without any viral assistance, e.g. by a totally plasmid-based method.

In a further aspect, the invention provides a method for generating in cultured cells infectious viral particles of an influenza A virus, said method comprising:
providing a first population of cells which are capable of supporting the growth of said virus and which have been modified to provide (a) the genomic vRNAs of said virus and (b) a nucleoprotein and RNA-dependent RNA polymerase whereby RNP complexes containing said genomic vRNAs are formed and said infectious viral particles are assembled, said genomic vRNAs being directly expressed in said cells under the control of a mammalian Pol I promoter or functional derivative thereof in the absence of a helper virus, and
wherein said cells do not produce interferon.

It will be appreciated that host cells modified for viral rescue as described above constitute a further aspect of the present invention. Viral particles produced by said first population of cells may be amplified by one or more further cellular infection steps, for example, employing cultured cells the same or different from said first population of cells. For use, the viral particles thus produced may be isolated.

Where the viral particles initially produced by the host cells are not attenuated, a conventional attenuation or viral killing step may be subsequently carried out, for example, prior to formulation for vaccine use. Viral particles generated in accordance with the invention may be recombinant viral particles capable of expressing a heterologous sequence. Such particles, if necessary after attenuation or killing as appropriate, may also be formulated for vaccine use or other therapeutic purpose.

In contrast to earlier helper virus-based rescue techniques for influenza virus, methods of the invention can easily be used for generation of infectious influenza virus of type A containing multiple mutations in several different genes at the same time. Such a method also enables easier and more direct production of reassortant influenza A virus containing vRNA segments derived from more than one parent virus. Conventionally, reassortant viruses are obtained by screening viral particles from a mixed viral infection of cells. A method of the invention is particularly advantageous for production of reassortant influenza A virus which are difficult to isolate by classic methods.

### Brief description of the figure

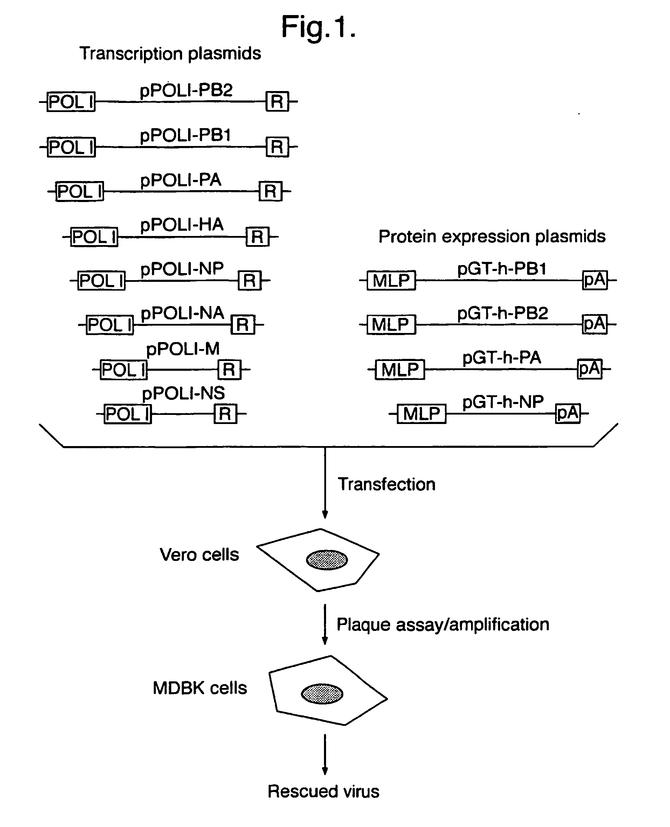

Figure 1 is a schematic representation of an embodiment of the invention, as further described in Examples 1 to 3, in which 12 plasmids for direct expression of the vRNA segments of an influenza A virus and expression of influenza A nucleoprotein and RNA-dependent RNA polymerase subuints are cotransfected into cultured Vero cells (African green monkey kidney cells). In this embodiment, MDBK (Madin-Darby bovine kidney) cells are employed for plaque assay and amplification of rescued viral particles. However, it will be appreciated that other cells which support growth of influenza A virus may equally be employed for plaque assay and amplification including Vero cells and MDCK (Madin-Darby canine kidney) cells. In Figure 1, POL I = truncated human RNA polymerase I promoter, R= genomic hepatitis virus ribozyme, MLP=adenovirus type 2 major late promoter linked to a synthetic sequence comprising the spliced tripartite leader sequence of human adenovirus type 2 and pA=polyadenylation sequence from SV40.

### Detailed description of the invention

It is preferred to employ expression vectors to directly express genomic vRNA segments of influenza A virus. These may be entirely wild-type vRNA segments or may include at least one non-wild type vRNA segment, e.g. a mutant vRNA segment having one or more nucleotide substitutions, insertions or deletions.

For example, at least one vRNA segment provided in the host cells may be a chimeric vRNA segment capable of expressing a sequence heterologous to the viral genome in target cells infected by the rescued virus. Such a heterologous sequence may encode a peptide or a polypeptide. It may alternatively encode a nucleic acid such as a ribozyme or anti-sense nucleic acid. The heterologous sequence may be provided on a vRNA segment additionally encoding a complete native viral protein as illustrated by the chimeric vRNA segment described in Example 8 or may be inserted into the coding sequence for a viral protein. For example, it is known that that the HA protein of influenza A can tolerate epitope-grafting in the antigenic site B. Methods for constructing such chimeric vRNA segments are reviewed, for example, in Muster and Garcia-Sastre, Ch. 9, Textbook of Influenza (1998) and Palese et al., Proc. Natl. Acad. Sci. USA (1996) 93, 11354-11358. Strategies for constructing chimeric influenza virus vRNA segments have also previously been described in Published International Patent Application WO 91/03552.

The vRNA segments provided in the host cells may additionally or alternatively incorporate one or more attenuating mutations. For example, the vRNA segments may be the vRNA segments of an influenza A virus having an attenuating base pair substitution in a pan-handle duplex promoter region, in particular, for example, the known attenuating base pair substitution of A for C and U for G at position 11-12' in the duplex region of the NA-specific vRNA (Fodor et al., J. Virol. (1998) 6283-6290). By using the rescue system of the invention, new attenuating mutations may be identified. As indicated above, where non-attenuated viral particles are initially produced by a method of the invention, attenuation or killing of the viral particles may, however, be subsequently achieved, for example, by classic methods.

Attenuated or killed viruses produced in accordance with the invention may subsequently be incorporated into a vaccine composition in conventional manner. Where such a virus has a chimeric vRNA segment as discussed above which encodes a foreign antigen, it may be formulated to achieve vaccination against more than one pathogen simultaneously. Attenuated recombinant viruses produced in accordance with the invention which possess a chimeric vRNA segment may also be designed for other therapeutic uses, e.g. an anti-tumour agent or gene therapy tool, in which case production of the virus will be followed by its incorporation into an appropriate pharmaceutical composition together with a pharmaceutically acceptable carrier or diluent.

As also indicated above, helper-virus free rescue in accordance with the invention is particularly favoured for generation of reassortant viruses, especially reassortant influenza viruses desired for vaccine use. For example, by means of viral rescue in accordance with the invention the HA and NA vRNA segments of an influenza virus, e.g. influenza A/PR8/34 which is recognized as suitable for human administration, may be readily substituted with the HA and NA vRNA segments of an influenza strain associated with an influenza infection epidemic. Such reassortant influenza viruses may, for example, be used for production of a killed influenza vaccine in conventional manner (see Examples 4 and 6).

The expression vectors employed may preferably be plasmids capable of replication in the chosen host cells. A separate expression vector may be provided for direct expression of each required vRNA segment or the corresponding cRNA. As already mentioned above, a second set of expression vectors may also be provided for each of the nucleoprotein and the individual RNA-dependent RNA polymerase subunits, e.g. the PB1, PB2 and PA subunits of an influenza virus RNA-dependent RNA polymerase. However, as also indicated above, alternatively a cell line may be employed which is capable of expressing one or more of these proteins in which case the second set of expression vectors may be reduced or even eliminated. Example 7 illustrates such a method for helper virus-free rescue of an influenza A virus employing a cell line stably expressing the nucleoprotein.

All the required expression vectors may preferably be introduced into the chosen host cells in a single cotransfection or cotransduction step. For this purpose, liposomal transfection may preferably be employed, for example using DOTAP liposomal transfection reagent (Boehinger Mannheim) or LipofectAMINE 2000 (Gibco BRL).

However, it will be appreciated that more than one vector transfer step may be carried out and other known means for introduction of vectors into mammalian cells employed, for example, electroporation, DEAE-dextran transfection, microparticle-bombardment and viral transduction, e.g. use of replication-defective retroviruses. Calcium phosphate preciptation is also particularly preferred (see Example 5). It may be chosen for example, to introduce into the host cells expression vectors for expression of the NP and polymerase proteins before the expression vectors for expression of the vRNA segments. The host cells into which the required vectors are introduced may be in a culture dish or cultured in other appropriate ways for vector transfection or transduction.

Expression of the vRNA segments or corresponding cRNAs will preferably be under the control of a promoter sequence derived from a mammalian RNA Pol I promoter. Particularly preferred for this purpose is the truncated human RNA Pol I promoter consisting of nucleotides -250 to -1 of the corresponding native promoter or a functional derivative thereof (Jones et al., Proc. Natl. Acad. Sci. USA (1988) 85, 669-673).Other promoters may, however, alternatively be employed, including, for example, a T7 RNA polymerase promoter. To ensure the correct 3' end of each expressed vRNA or cRNA, each vRNA or cRNA expression vector will incorporate a ribozyme sequence or appropriate terminator sequence downstream of the RNA coding sequence. This may be, for example, the hepatitis delta virus genomic ribozyme sequence or a functional derivative thereof Alternatively, for example, a Pol I terminator may be employed (Neumann et al., Virology (1994) 202, 477-479). The RNA expression vectors may be constructed in the same manner as the vRNA expression vectors described in Pleschka et al., J. Virol.(1996) 70, 4188- 4192.

Where one or more protein expression vectors are required to express viral proteins for RNP complex formation, these will preferably express the required viral protein(s) homologous to the desired virus. Expression of the nucleoprotein and RNA-dependent polymerase subunits may preferably, for example, be under the control of a regulatory sequence comprising the adenovirus 2 major late promoter linked to the spliced tripartite leader sequence of human adenovirus type 2, as described by Berg at al., BioTechniques, 14, 972-978, or a functional derivative of said regulatory sequence. However, alternative promoter sequences operative in mammalian cells may possibly be substituted such as, for example, another viral promoter such as, for example, the human cytomegalovirus (CMV) immediate-early promoter or a T7 polymerase promoter. Appropriate plasmids for expression of the NP and polymerase subunits may be constructed, for example, starting from the plasmid pGT-h as also described in the above-noted paper of Berg et al.

The invention will be further described below with specific reference to rescue of influenza A viruses. For the purpose of influenza A rescue by the strategy of the invention utilising plasmids which directly express the required vRNAs, it has been found favourable, for example, to use Vero (African green monkey kidney) cells, although other cells which support growth of influenza viruses may be employed, for example, preferably 293T human embryonic kidney cells (293T cells are disclosed herein for technical reference). Such cells may be transfected preferably on the surface of an appropriate culture dish.

It is known that Vero cells are deficient in interferon expression (Diaz et al., Proc. Natl. Acad. Sci. USA (1998) 85 5259-5263), which might be a factor in attaining good viral rescue. Hence, it is extrapolated that Vero cells and other cells deficient in interferon activity or response which will support growth of segmented negative-strand RNA viruses are useful in the practice of the invention.

Appropriate amounts and ratios of vectors for carrying out a method of the invention may be determined by routine experimentation. As guidance, in the case of liposomal transfection or calcium preciptation of plasmids into the host cells, it is envisaged that each plasmid may be employed at a few µgs, e.g 1 to 10 µg, for example, diluted to a final total DNA concentration of about 0.1 µg/ml prior to mixing with transfection reagent in conventional manner. It may be preferred to use vectors expressing NP and/or RNA-dependent RNA polymerase subunits at a higher concentration than those expressing vRNA segments.

In accordance with certain aspects, the present specification also provides a method for generating in cultured cells infectious viral particles of an influenza A virus said method comprising:
(i) providing a population of cells which are capable of supporting the growth of said virus and which are modified so as to be capable of providing (a) the genomic vRNAs of said virus in the absence of a helper virus and (b) a nucleoprotein and RNA-dependent RNA polymerase whereby RNP complex or complexes containing said genomic vRNAs can be formed and said viral particles can be assembled, said genomic RNAs being directly expressed in said cells under the control of a mammalian Pol I promoter or a functional derivative thereof, e.g. the truncated human Pol I promoter as previously noted above and
(ii) culturing said cells whereby said viral particles are produced.

Such virus-producing cells may, for example, preferably be Vero cells or other cells deficient in interferon activity or response which will support the growth of a segmented negative-strand RNA virus. All or some of the coding sequences for said genomic vRNAs, nucleoprotein and RNA-dependent RNA polymerase may be provided in the chose host cells by introducing expression vectors into the cells.

The following examples illustrate the invention with reference to helper virus-free rescue of an influenza A virus. However, as previously indicted above, the disclosure can be applied to other segmented negative-strand RNA virus, especially, for example, influenza viruses of other types.

### Examples

### Example 1

### Preparation of plasmids encoding the vRNA segments of an influenza A virus

Eight plasmids each expressing a different vRNA segment of influenza A/WSN/33 were used (pPOL1-PB2-RT, pPOL1-PB1-RT, pPOL1-PA-RT, pPOL1-HA-RT, pPOLI-NP-RT, pPOL1-NA-RT, pPOL1-M-RT and pPOL1-NS-RT). These were pUC19 or pUC18-based plasmids analogous in structure to the model vRNA segment encoding plasmid, pPOL1-CAT-RT, described in Pleschka et al. (1996) J. Virol. 70, 4183-4192, apart from substitution of the cDNA encoding the vRNA CAT reporter gene segment (an open reading frame for chloramphenicol acetytransferase in negative polarity flanked by the non-coding regions of the NS-encoding vRNA segment of influenza A/WSN/33) by a cDNA encoding a native vRNA segment of influenza A/WSN/33. In each of these plasmids, a truncated human RNA Pol I promoter (positions -250 to -1) was fused to the end of the vRNA segment encoding cDNA to ensure the correct 5' end of the transcribed vRNA. Also provided in each of the vRNA segment encoding plasmids was the sequence of the hepatitis delta virus genomic ribozyme to also ensure the correct 3' end of the transcribed vRNA.

Samples of influenza A/WSN/33 for preparation of the cDNA inserts of the above-described plasmids are obtainable, for example, from the W.H.O. Collaborating Centre, Division of Virology, National Institute for Medical research, London, U.K.)

### Example 2

### Preparation of plasmids for expression of the PB1, PB2, PA and NP proteins of influenza A/WSN/33.

4 expression plasmids (pGT-h-PB1, pGT-h-PB2, pGT-h-PA and p-GT-h-NP) were additionally prepared each capable of expressing a different protein selected from the required PB1, PB2, PA and NP proteins under the control of the adenovirus 2 major late promoter linked to a synthetic sequence comprising the spliced tripartite leader sequence of human adenovirus type 2. This promoter was previously reported to give high-level expression of proteins in cells adapted to serum-free suspension culture (Berg et al.(1993) BioTechniques, 14, 972-978). The pGT-h set of protein expression plasmids was constructed by inserting the open reading frames for the PB1, PB2, PA and NP proteins into the Bcl I cloning site of the pGT-h plasmid (Berg et al. (1993), *ibid.*)

The expression plasmids encoding the viral nucleoprotein and 3 protein subunits of the viral RNA-dependent RNA polymerase were cotransfected into human 293 cells or Vero cells with the expression plasmid pPOL1-CAT-RT. In both the transfected human 293 cells and Vero cells, CAT activity could be detected. Vero cells were chosen for helper-virus free generation of influenza A/WSN/33 from transfected vRNA segments as further described below since they support better growth of influenza A/WSN/33 than human 293 cells (about one log difference in maximum viral titre).

### Example 3

### Helper virus free rescue of influenza A/WSN/33

For viral rescue, near-confluent Vero cells in 8.5 cm diameter dishes (about 10⁷ cells covering about 90% of the dish) were cotransfected with the four protein expression plasmids and the eight vRNA transcription plasmids described above. For this cotransfection step, 5 µg of each of the polymerase protein expression plasmids, 10 µg of the NP-expressing plasmid and 3 µg of each of the 8 vRNA-encoding plasmids were diluted to a concentration of 0.1 µg/µl in 20mM Hepes buffer (pH 7.5).The DNA solution was added to diluted DOTAP liposomal transfection reagent (Boehringer Mannheim) containing 240 µl of DOTAP and 720 µl of 20mM Hepes buffer (pH 7.5). The transfection mixture was incubated at room temperature for 15 mins and then mixed with 6.5 ml of Minimal Essential Medium (MEM) containing 0.5% fetal calf serum (FCS), 0.3% bovine serum albumin (BSA), penicillin and streptomycin. This mixture was added to the Vero cells washed with PBS. After 24 hours, the transfection medium was removed from the cells and replaced with 8 ml of fresh medium (MEM) containing 0.5% FCS, 0.3% BSA, penicillin and streptomycin. The transfected Vero cells were cultured for at least 4 days after transfection. Every day, the medium from the transfected cells was collected and assayed for the presence of influenza virus by plaquing a 0.5 ml aliquot on MDBK cells in conventional manner. The rest of the medium was transferred into 75 cm² flasks of subconfluent MDBK cells for amplification of any rescued virus. The original transfected cells were further incubated after adding 8 ml of fresh medium.

This procedure resulted in the recovery of infectious influenza virus on day 4 post transfection. About 10 to 20 plaque-forming viral particles were obtained from a 8.5 cm dish containing approximately 10⁷ cells. The rescued virus showed a specific property characteristic of influenza A/WSN/33 virus, i.e. it formed plaques on MDBK cells in the absence of trypsin. The plaques formed by the rescued virus were comparable in size to those formed by a control authentic A/WSN/33 virus sample grown on the same MDBK cells.

To confirm that the viral plaques observed on the MDBK cells treated with virus harvested from the culture medium of transfected cells were derived from the cloned cDNAs, genetic tags were introduced into two of the 8 vRNA segment cDNAs. A cDNA was constructed encoding an HA vRNA segment with a mutation of 6 nucleotides near the 3' end of the segment. Nucleotides 31 to 35 from the 3' end (3'-UUUUG-5') were replaced with 3'-AAAAC-5' resulting in amino acid substitution at amino acid 4(K→F) and at amino acid 5 (L→V) near the N-terminus of HA within the signal peptide. In addition, a silent C→U mutation was created at nucleotide 40. These changes introduced several new restriction sites, including a unique SpeI site. The cDNA encoding the NA segment was mutated to encode an NA segment containing two silent mutations at nucleotides 1358 and 1360 so as to introduce a new unique SacI restriction site (Pleschka et al., J. Virol (1996) 70, 4188-4192).

Medium from MDBK cells infected with the rescued transfectant virus was used to isolate vRNA. 100 µl of the medium was treated with 5 u of RNase-free DNase to remove any residual plasmid DNA carried over. After 15mins at 37°C, vRNA was isolated using the RNeasy Mini Kit (Qiagen). Short regions of the HA and NA vRNAs expected to contain the genetic tags were amplified by RT-PCR and then analysed by digestion with SpeI and SacI restriction enzymes, respectively. As a control, the same regions of the HA and NA segments were amplified from vRNA isolated from authentic influenza A/WSN/33 virus using the same RT-PCR primers.

The PCR products obtained from the rescued virus and the control virus were the same size. Those originating from the HA and NA segments of the rescued virus could be digested with SpeI and SacI respectively. However, the PCR products corresponding to the control virus were, as expected, not digested by the same enzymes. The omission of reverse transcriptase in control RT-PCR reactions resulted in no visible PCR products.

### Comments

The studies described above show that it is possible to rescue an influenza A virus by cotransfecting 8 transcription plasmids for the individual vRNA segments and 4 expression plasmids encoding the required NP, PB1, PB2 and PA proteins into Vero cells in the absence of any helper virus.

It is highlighted that in the above-noted studies, influenza virus was generated by expressing negative sense vRNA segments. This seems to contradict some earlier studies which emphasised the importance of using positive strand RNA for rescuing negative strand RNA viruses, including Bunyamwera virus whose genome is in 3 segments (Schnell et al. (1994) EMBO J., 13, 4195-4203; Roberts and Rose (1998) Virology 247, 1-6; Bridgen and Elliot (1996) 93, 15400-15404). However, more recent successful recoveries of non-segmented negative-strand RNA viruses from negative sense RNA have been reported (Kato et al.(1996) Genes Cells 1, 569-579; Durbin et al. (1997) Virology 235,323-332).

In a protocol of the invention as illustrated above, at early stages post-transfection positive-sense mRNA from the 4 protein expression plasmids coexists with naked negative-sense genomic vRNA transcribed from the transcription plasmids. Thus, double-stranded RNA may form. Formation of such double-stranded RNA in human cells could possibly lead to the induction of interferon-mediated antiviral responses and consequently to suppression of the growth of any rescued virus. However, such interferon-induction is obviated as a problem in Vero cells since such cells are deficient in interferon expression.

### Example 4

### Helper virus free rescue of A/PR/8/34 influenza (Cambridge variant)

In order to rescue A/PR/8/34 entirely from recombinant DNA,12 plasmids were generated. The 12 plasmids are analogous to those described for the rescue of A/WSN/33 virus (see Examples 1 and 2 above), with a few modifications. The 8 plasmids required for the synthesis of the 8 vRNA segments, by cellular RNA Polymerase 1, have a murine rDNA terminator sequence (GenBank, accession number M12074) instead of the hepatitis delta virus ribozyme to generate the exact 3' end of the vRNA segments. The 4 protein expression plasmids for the A/PR/8/34 polymerase subunits (PB1, PB1, PA) and the nucleoprotein (NP) are based on the commercially available pcDNA3 (Invitrogen, Catalogue No. V790-20), which has a cytomegalovirus (CMV) promoter and a bovine growth hormone (BGH) poly(A) site.

### Construction of the plasmid pPolISapIT

In order to allow easy cloning of the 8 vRNA segments, a new basic cloning vector, pPolISapIT, was constructed. In this new construct, the murine rDNA terminator sequence (positions +572 to +715) is positioned downstream of the Pol I promoter. The Pol I promoter and terminator sequences are separated by a 24 bp linker sequence (5'-AGAAGAGCCAGATCTGGCTCTTCC-3'), containing SapI restriction sites.

Plasmid pPolISapIT was derived from pPolI-CAT-RT (originally described in Pleschka et al., J. Virol. 70, 4188-4192, 1996). A DNA fragment containing a region of the murine rDNA terminator sequence (positions +335 to +715, GenBank accession number M12074) was inserted into the SalI site of pPolI-CAT-RT to generate pPolI-CAT-T. Subsequently,by using an inverse PCR technique, the CAT gene, the ribozyme and part of the murine rDNA terminator sequence (positions +335 to +571) were deleted from pPolI-CAT-T. At the same time, the 24 bp linker sequence as given above was introduced through the PCR primers between the Pol I promoter and the murine rDNA terminator sequence.

### Construction of the vRNA expression vectors

cDNA was generated by RT-PCR from vRNA isolated from influenza A/PR/8/34 virus (Cambridge variant) using PCR primers with SapI overhangs. After SapI digestion, the PCR products were cloned into pPolISapIT digested with SapI.

### Viral rescue

Cotransfection of the 12 plasmids into Vero cells using DOTAP transfection reagent and following the protocol given in Example 3 (see also Fodor et al., J. Virol. (November 1999) 73, 9679-9682) resulted in the rescue of infectious influenza A/PR/8/34 particles on day 4 post-transfection. Plaque assays and viral amplification were performed on MDCK cells in the presence of 0.5 µg/ml trypsin.

### Comments

These results demonstrate that influenza A/PR8/34 virus can be successfully rescued by the helper virus-free method of the invention. This is of particular interest since influenza A/PR8/34 is known to be avirulent to humans (Beare et al. (1975) Trials in man with live recombinants made from A/PR8/34 (HON1) and wild H3 N2 influenza viruses, Lancet (ii) 729-732) whereas influenza A/WSN/33 is considered unsuitable for administration to humans because of its known neurotropism in mice. It is thus proposed that influenza A/PR8/34, in a suitably attenuated form, would be suitable as a parent virus for live vaccine development. For example, helper virus-free viral rescue in accordance with the invention could be used to generate an attenuated reassortant virus starting with expression vectors for the vRNAs of influenza A/PR8/34 apart from substitution of the HA and NA genomic segments of A/PR8/34 virus with the HA and NA genomic segments of an influenza strain associated with an influenza infection epidemic. Further exemplification of use of helper-virus free viral rescue in accordance with the invention to generate reassortant influenza viruses is given in Example 6 below.

### Example 5

### Improved protocols for the helper virus free rescue of influenza A/WSN/33

The 4 protein expression plasmids specified in Example 2 were replaced with the protein expression plasmids specified in Example 4 derived from pcDNA3. Using these four protein expression plasmids together with the eight vRNA transcription plasmids specified in Example 1 (see also Fodor et al., J. Virol. (1999) 73. 9679-9682) in the 3 protocols set out below, between 100-10,000 plaque-forming viral particles from 10⁶ cells were obtained on day 2 post-transfection This is at least 100 times more virus than obtained by the transfection studies reported in Example 3.

### Protocol (a): Transfection of 293T cells using "LipofectAMINE 2000" transfection reagent

1 µg of each of the 12 plasmids were combined and the volume adjusted to 50 µl by adding OPTIMEM medium (Gibco BRL). In a polystyrene tube, 12 µl of LipofectAMINE 2000 (Gibco BRL, Cat. No. 11 668-027) and 238 µl of OPTIMEM medium were combined and the mixture incubated for 5 minutes at room temperature. The DNA mixture was then added drop-wise into the diluted LipofectAMINE 2000 transfection reagent. After incubating the DNA-Lipofectamine mixture at room temperature for about 20 minutes, the mixture was added drop-wise into a 293T cell suspension (about 10⁶ cells in 1 ml of DMEM containing 10% FCS without antibiotics). At about 16-24 hours post transfection, the transfection mixture was removed and replaced with 1 ml of DMEM containing 0.5% FCS, 0.3% BSA, penicillin and streptomycin. 24-48 hours later, rescued virus was screened for by plaquing 100 µl of the medium from the transfected 293 T cells on MDBK cells and by passaging the rest of the medium on a 25 cm² semiconfluent MDBK flask. 1 ml of DMEM containing 0.5% FCS, 0.3% BSA penicillin and streptomycin was added to the transfected 293T cells and incubation continued for another 2 to 3 days before repeating the plaquing and amplification on MDBK cells.

### Protocol (b): Transfection of 293T cells using calcium phosphate precipitation

For transfection using calcium phosphate precipitation, 1 µg of each of the 12 plasmids was combined and the plasmid mixture added to 250 µl 2x HEBS buffer (40 mM Hepes, 280 mM NaCl,10 mM KCl, 2 mM Na₂HPO₄,10 mM glucose, pH 7.05). Then 250 µl of 250 mM CaCl₂ was added and the contents of the tube mixed vigorously. After 20-30 mins at room temperature, the precipitate was mixed with 1 ml of DMEM containing 10% FCS, penicillin and streptomycin and added to a 293T cell suspension (about 10⁶ cells in 1 ml of DMEM containing 10% FCS without antibiotics). At about 16-24 hours post transfection, the transfection mixture was removed and replaced with 1 ml of DMEM containing 0.5% FCS, 0.3% BSA, penicillin and streptomycin. 24-48 hours later, rescued virus was screened for as in protocol (a) above.

### Protocol (c): Transfection of Vero cells using DOTAP transfection reagent

1 µg of each of the 12 plasmids was combined and the volume adjusted to 120 µl by adding 20 mM hepes (pH 7.5) to give a DNA concentration of about 0.1 µg/µl. The DNA solution was then added to diluted DOTAP transfection reagent (Boehringer) containing 60 µl of DOTAP and 200 µl of 20 mM Hepes (pH 7.5) in a polystyrene tube. After incubation of the DNA-DOTAP mixture at room temperature for about 15-20 minutes, the mixture was added drop-wise into a Vero cell suspension (about 10⁶ cells in 1 ml of MEM containing 10% FCS, penicillin and streptomycin). At about 16-24 hours post transfection, the transfection mixture was removed and replaced with 1 ml of MEM containing 0.5% FCS, 0.3% BSA, penicillin, and streptomycin. 24-48 hours later, rescued virus was screened for by plaquing 100 µl of the medium from the transfected Vero cells on MDBK cells and by passaging the rest of the medium on a 25 cm² semiconfluent MDBK flask. 1 ml of MEM containing 0.5% FCS, 0.3% BSA, penicillin, and streptomycin was added to the transfected Vero cells and incubation continued for another 2 to 3 days before repeating the plaquing and amplification on MDBK cells.

### Example 6

### Helper virus free rescue of reassortant influenza viruses

Plasmid-based rescue in accordance with the invention has been successfully used to generate reassortant influenza viruses. The following reassortant viruses were generated:
(i) A/WSN/33 with the PA segment derived from A/PR/8/34
(ii) A/WSN/33 with the NP segment derived from A/PR/8/34
(iii) A/WSN/33 with the M segment derived from A/PR/8/34
(iv) A/WSN/33 with the PB2 segment derived from A/FPV/Dobson/34

These examples demonstrate the utility of the helper virus free method for isolating reassortants. Reassortant viruses based on A/PR8/34 (or other suitable strains) are required for the production of conventional killed vaccines because they grow to high titre in embryonated chicken eggs - used in the commercial production of killed influenza vaccines. As previously indicated above, an important application of helper virus free viral rescue in accordance with the invention is thus seen to be easier and more direct isolation of reassortant viruses than by the classic method of isolating reassortants from a mixed infection of cells with two live viruses. Importantly, using a method of the invention, the need to screen many potential reassortants before the required one is isolated is obviated.

### Example 7

### Helper virus free rescue of influenza A/WSN/33 on EcR-293 cells

Rescue of influenza A/WSN/33 has been achieved on EcR-293NP cells, a cell line stably expressing influenza NP, by transfecting 11 plasmids.

EcR-293NP cells were derived from the commercially available cell line EcR-293 (Invitrogen, Catalogue No. R650-07) which constitutively expresses the VgEcR and RXR. subunits of the ecdysone receptor. Influenza NP expression in such cells is inducible in response to ponasterone A. The same protocol was used as specified in Example 5(a) employing LipofectAMINE 2000 transfection reagent except pcDNA-NP was omitted, since the NP protein for the initial encapsidation of the vRNA segments was provided by the EcR-293NP cells.

### Example 8

### Helper virus free rescue of a recombinant influenza virus expressing a foreign antigen

The plasmid pPOL1-E6N18-2A-NA was generated capable of expressing a chimeric vRNA segment based on the NA vRNA segment of influenza A/WSN/33 virus. The modified vRNA coding sequence was inserted between sequences corresponding to a truncated human Pol I promoter and hepatitis delta virus ribozyme as for preparation of the Pol I-expression plasmids described in Example 1. The resultant chimeric gene contained a long open reading frame (ORF) encoding the first 88 amino acids of the E6 protein of human papillomavirus 18 (HPV 18), followed by 17 amino acids corresponding to the self-cleavage motif of the 2A protease of foot-and-mouth-disease virus (FMDV), followed by the amino acid sequence of the NA of influenza A/WSN/33. The coding region was flanked by the non-coding regions of the NA gene of A/WSN/33 virus. In this way, a chimeric influenza virus gene was generated encoding a polyprotein that undergoes self-cleavage, resulting in the generation of an HPV-derived polypeptide and the NA protein. A similar strategy for the expression of foreign antigens by influenza virus vectors generated by classical RNP-transfection has previously been described (T. Muster and A. Garcia-Sastre, Genetic manipulation of influenza viruses, in Textbook of Influenza, K.G. Nicholson, R.G. Webster & A.J. Hay, eds., pp. 93-106 (1998), Blackwell Science Ltd, Oxford, UK.)

The recombinant influenza virus vector expressing the HPV18-derived antigen was generated by co-transfecting into 293T cells pPOL1-E6N18-2A-NA together with 7 PolI-expression vectors encoding wild-type viral RNAs, i.e. PB2, PB1, PA, HA, NP, M and NS as described in Example 1 and the 4 PolII-expression vectors encoding the PB2, PB 1, PA and NP proteins as described in Example 5. The rescued virus had the correct nucleotide sequence as confirmed by sequence analysis of its NA-specific viral RNA.

## Claims

1. A method for generating infectious viral particles of an influenza A virus, said method comprising:
introducing into cultured cells expression vectors that express in said cells the complete genomic vRNA segments of said virus, wherein said cells support growth of said virus, said cells also providing a nucleoprotein and RNA-dependent RNA polymerase whereby RNP complexes containing the genomic vRNA segments of said virus are formed and said infectious viral particles are produced by said cells in the absence of a helper virus and wherein said cells do not produce interferon.

2. A method as claimed in claim 1 wherein one or more further expression vectors are employed in said cells to express one or more proteins selected from said nucleoprotein and the subunits of said RNA-dependent RNA polymerase.

3. A method as claimed in claim 1 or claim 2 wherein a cell line is employed which is capable of expressing one or more of said nucleoprotein and the subunits of said RNA-dependent RNA polymerase.

4. A method as claimed in any one of claims 1 to 3 wherein said virus is a reassortant virus having vRNA segments derived from more than one parent virus.

5. A method as claimed in any one of claims 1 to 4 wherein said cells are Vero cells.

6. A method as claimed in any one of claims 1 to 5 wherein said expression vectors defined in claim 1 express genomic vRNA segments of said virus.

7. A method as claimed in any one of claims 1 to 6 further comprising amplifying said formed viral particles by one or more subsequent cellular infection steps employing the same or different type of cells.

8. A method as claimed in any one of claims 1 to 7 which further comprises isolating infectious viral particles.

9. A method as claimed in any one of claims 1 to 8 which further comprises a viral attenuation or killing step.

10. A method as claimed in any one of claims 1 to 9 wherein all the required expression vectors are cotransfected into said cells using a liposomal transfection reagent, calcium phosphate precipitation or electroporation.

11. A method as claimed in any one of claims 1 to 10 wherein said expression vectors are all plasmids.

12. A method as claimed in any one of claims 1 to 11 wherein said expression vectors defined in claim 1 consist of separate expression vectors for expression of each vRNA segment of said virus.

13. A method as claimed in any one of claims 1 to 12 wherein the expression of each vRNA segment is under the control of a promoter sequence derived from a mammalian PolI promoter.

14. A method as claimed in claim 13 wherein said promoter sequence is a truncated human PolI promoter sequence consisting of nucleotides -250 to -1 of the corresponding native promoter or a functional derivative thereof.

15. A method as claimed in any one of claims 1 to 14 wherein the coding sequence for each vRNA segment in said expression vectors is followed by a ribozyme sequence or transcription terminator that produces a correct 3' end of each said RNA.

16. A method as claimed in claim 2 wherein expression of one or more viral proteins from said further expression vectors is under the control of a regulatory sequence selected from the adenovirus 2 major late promoter linked to the spliced tripartite leader sequence of human adenovirus type 2 or the human cytomegalovirus immediate-early promoter, or a functional derivative of said regulatory sequence.

17. A method as claimed in any one of claims 1 to 16 which further comprises incorporating an attenuated or killed virus into a vaccine composition.

18. A method as claimed in any one of claims 1 to 17 wherein said virus has at least one vRNA segment capable of directing expression of a sequence heterologous to said virus In target cells infected by said virus.

19. A method as claimed in claim 18 wherein said sequence heterologous to said virus encodes an antigenic peptide or antigenic polypeptide and which further comprises incorporating said virus into a vaccine composition.

20. Modified cells as produced in claim 1 or claim 5.

21. A method of producing infectious viral particles of a segmented negative-strand virus which comprises culturing modified cells as claimed in claim 20.

22. A method for generating in cultured cells infectious viral particles of an influenza A virus, said method comprising:
providing a first population of cells which are capable of supporting the growth of said virus and which have been modified to provide (a) the genomic vRNAs of said virus and (b) a nucleoprotein and RNA-dependent RNA polymerase whereby RNP complexes containing said genomic vRNAs are formed and said infectious viral particles are assembled, said genomic vRNAs being directly expressed in said cells under the control of a mammalian Pol I promoter or functional derivative thereof in the absence of a helper virus, and wherein said cells do not produce interferon.

23. The method of claim 22 further comprising amplifying said assembled viral particles by one or more subsequent cellular infection steps employing the same or different type of cells.

24. A method as claimed claim 22 or claim 23 which further comprises isolating infectious viral particles.

25. A method as claimed in any one of claims 22 to 24 which further comprises an attenuation or viral killing step.

26. A method as claimed in any one of claims 22 to 25 which further comprises incorporating attenuated or killed viral particles into a vaccine composition.

27. A method as claimed in any one of claims 22 to 25 wherein said virus has at least one vRNA segment capable of directing expression of a sequence heterologous to said virus in target cells infected by said virus and which further comprises incorporating said virus, if appropriate after attenuation or killing, into a pharmaceutical composition together with a pharmaceutically acceptable carrier or diluent.

28. A method as claimed in any one of claims 22 to 27 wherein said cells are Vero cells.

29. Modified cells as produced in claim 22 or claim 28.

30. A method of producing infectious viral particles of a segmented negative-strand virus which comprises culturing modified cells as claimed in claim 29.

31. The method of any one of claims 7 to 15 and 17 to 19 wherein the cells used for amplification are MDBK cells or MDCK cells.

32. The method of any one of claims 23 to 28 wherein the cells used for amplification are MDBK cells or MDCK cells.

33. The method of claims 1 to 19, 21 to 28 or 30 to 32 wherein the cells are EcR-293NP cells.

## Patentansprüche

1. Verfahren zum Erzeugen von infektiösen Viruspartikeln eines Influenza A Viruses, welches Verfahren umfasst:
Einführen von Expressionsvektoren in kultivierte Zellen, die in diesen Zellen die vollständigen genomischen vRNA-Segmente dieses Virus exprimieren und wobei diese Zellen das Wachstum des Virus unterstützen und diese Zellen auch ein Nukleoprotein und eine RNA-abhängige RNA-Polymerase bereitstellen, wodurch RNP-Komplexe gebildet werden, die die genomischen vRNA-Segmente dieses Virus enthalten, und die infektiösen Viruspartikel durch diese Zellen in Abwesenheit eines Helfervirus erzeugt werden und wobei diese Zellen kein Interferon erzeugen.

2. Verfahren nach Anspruch 1, bei dem ein oder mehrere weitere Expressionsvektoren in diesen Zellen eingesetzt werden, um ein oder mehrere Proteine zu exprimieren, die aus dem Nukleoprotein und den Untereinheiten der RNA-abhängigen RNA-Polymerase ausgewählt sind.

3. Verfahren nach Anspruch 1 oder Anspruch 2, bei dem eine Zell-Linie eingesetzt wird, die in der Lage ist, ein oder mehrere des Nukleoproteins und der Untereinheiten der RNA-abhängigen RNA-Polymerase zu exprimieren.

4. Verfahren nach irgendeinem der Ansprüche 1 bis 3, bei dem das Virus ein reassortantes Virus ist, das über vRNA-Segmente verfügt, die von mehr als einem parentalen Virus abgeleitet sind.

5. Verfahren nach irgendeinem der Ansprüche 1 bis 4, bei dem die Zellen Vero-Zellen sind.

6. Verfahren nach irgendeinem der Ansprüche 1 bis 5, bei dem die in Anspruch 1 definierten Expressionsvektoren genomische vRNA-Segmente dieses Virus exprimieren.

7. Verfahren nach einem der Ansprüche 1 bis 6, ferner umfassend das Amplifizieren der gebildeten Viruspartikel durch einen oder mehrere nachfolgende zelluläre Infektionsschritte, bei dem der gleiche oder verschiedene Zelltypen eingesetzt werden.

8. Verfahren nach irgendeinem der Ansprüche 1 bis 7, ferner umfassend das Isolieren infektiöser Viruspartikel.

9. Verfahren nach irgendeinem der Ansprüche 1 bis 8, ferner umfassend einen Schritt der viralen Attenuation oder Abtötung.

10. Verfahren nach irgendeinem der Ansprüche 1 bis 9, bei dem alle erforderlichen Expressionsvektoren durch Verwendung eines liposomalen Transfektionsreagens, Calciumphosphat-Ausfällung oder Elektroporation in die Zellen cotransfiziert werden.

11. Verfahren nach irgendeinem der Ansprüche 1 bis 10, bei dem die Expressionsvektoren alle Plasmide sind.

12. Verfahren nach irgendeinem der Ansprüche 1 bis 11, bei dem die in Anspruch 1 definierten Expressionsvektoren aus separaten Expressionsvektoren zur Expression jedes vRNA-Segments des Virus bestehen.

13. Verfahren nach irgendeinem der Ansprüche 1 bis 12, bei dem die Expression jedes vRNA-Segments unter der Kontrolle einer Promotorsequenz steht, die von einem Pol 1-Promotor eines Säugers abgeleitet ist.

14. Verfahren nach Anspruch 14, bei dem die Promotorsequenz eine trunkierte humane Pol I-Promotorsequenz ist, die aus Nukleotiden -250 bis -1 des entsprechenden nativen Promotors oder einem funktionellen Derivat davon besteht.

15. Verfahren nach irgendeinem der Ansprüche 1 bis 14, bei dem der kodierenden Sequenz für jedes vRNA-Segment in den Expressionsvektoren eine Ribozym-Sequenz oder Transkriptionsterminator folgen, der an jeder RNA ein korrektes 3'-Ende erzeugen.

16. Verfahren nach Anspruch 2, bei dem die Expression von einem oder mehreren viralen Proteinen aus den weiteren Expressionsvektoren unter der Kontrolle einer regulatorischen Sequenz steht, die ausgewählt ist aus dem Adenovirus 2-Major Late Promotor, verknüpft mit der gespleißten, Tripartite-Leadersequenz von humanem Adenovirus Typ 2 oder dem humanen Cytomegalovirus-Immediate-Early Promotor oder einem funktionellen Derivat dieser regulatorischen Sequenz.

17. Verfahren nach einem der Ansprüche 1 bis 16, ferner umfassend das Inkorporieren eines attenuierten oder abgetöteten Virus in einer Vakzin-Zusammensetzung.

18. Verfahren nach einem der Ansprüche 1 bis 17, bei dem das Virus mindestens ein vRNA-Segment hat, das in der Lage ist, die Expression einer zu dem Virus heterologen Sequenz in Targetzellen zu dirigieren, die mit diesem Virus infiziert sind.

19. Verfahren nach Anspruch 18, bei dem die zu dem Virus heterologe Sequenz ein antigenes Peptid oder antigenes Polypeptid codiert und welches Verfahren ferner das Inkorporieren dieses Virus in eine Vakzin-Zusammensetzung umfasst.

20. Modifizierte Zellen, wie in Anspruch 1 oder Anspruch 5 hergestellt.

21. Verfahren zum Erzeugen infektiöser Viruspartikel eines segmentierten Minus-Strang-Virus, welches Verfahren das Kultivieren modifizierter Zellen nach Anspruch 20 umfasst.

22. Verfahren zum Erzeugen infektiöser Viruspartikel eines Influenza A Viruses in kultivierten Zellen, welches Verfahren umfasst:
Bereitstellen einer ersten Population von Zellen, die in der Lage sind, das Wachstum des Virus zu unterstützen und die modifiziert worden sind, um (a) die genomischen vRNA's des Virus und (b) ein Nukleoprotein und RNA-abhängige RNA-Polymerase bereitzustellen, wodurch RNP-Komplexe, die diese genomischen vRNA's enthalten, gebildet werden und die infektiösen Viruspartikel zusammengefügt werden, wobei die genomischen vRNA's direkt in diesen Zellen unter der Kontrolle eines Pol I-Promotors eines Säugers oder eines funktionellen Derivats davon in Abwesenheit eines Helfervirus exprimiert werden und wobei die Zellen kein Interferon erzeugen.

23. Verfahren nach Anspruch 22, ferner umfassend das Amplifizieren der zusammengefügten Viruspartikel mit Hilfe eines oder mehrerer nachfolgender zellulärer Infektionsschritte unter Einsatz desselben oder eines anderen Zelltyps.

24. Verfahren nach Anspruch 22 oder Anspruch 23, ferner umfassend das Isolieren von infektiösen Viruspartikeln.

25. Verfahren nach irgendeinem der Ansprüche 22 bis 24, ferner umfassend einen Attenuierungs- oder viralen Abtötungsschritt.

26. Verfahren nach irgendeinem der Ansprüche 22 bis 25, das ferner das Inkorporieren von attenuierten oder abgetöteten Viruspartikeln in eine Vakzin-Zusammensetzung umfasst.

27. Verfahren nach irgendeinem der Ansprüche 22 bis 25, bei dem das Virus mindestens ein vRNA-Segment hat, das in der Lage ist, die Expression einer zu dem Virus heterologen Sequenz in Targetzellen zu dirigieren, die mit diesem Virus infiziert sind, wobei das Verfahren ferner das Inkorporieren des Virus, wenn geeignet nach der Attenuation oder nach dem Abtöten, in eine pharmazeutische Zusammensetzung gemeinsam mit einem pharmazeutisch akzeptablen Träger oder Verdünnungsmittel umfasst.

28. Verfahren nach irgendeinem der Ansprüche 22 bis 27, bei dem die Zellen Vero-Zellen sind.

29. Modifizierte Zellen wie in Anspruch 22 oder Anspruch 28 hergestellt.

30. Verfahren zum Herstellen infektiöser Viruspartikel eines segmentierten Minus-Strang-Virus, das das Kultivieren modifizierter Zellen nach Anspruch 29 umfasst.

31. Verfahren nach irgendeinem der Ansprüche 7 bis 15 und 17 bis 19, bei dem die zur Amplifikation verwendeten Zellen MDBK-Zellen oder MDCK-Zellen sind.

32. Verfahren nach irgendeinem der Ansprüche 23 bis 28, bei dem die zur Amplifikation verwendeten Zellen MDBK-ZelIen oder MDCK-ZelIen sind.

33. Verfahren nach den Ansprüchen 1 bis 19, 21 bis 28 oder 30 bis 32, bei dem die Zellen EcR-293NP-Zellen sind.

## Revendications

1. Procédé destiné à générer des particules virales infectieuses du virus de la grippe A, ledit procédé comprenant le fiait :
d'introduire dans des cellules cultivées des vecteurs d'expression qui expriment dans lesdites cellules les segments d'ARNv génomiques complets dudit virus, où lesdites cellules soutiennent la croissance dudit virus, lesdites cellules fournissant également une nucléoprotéine et une ARN polymérase dépendante de l'ARN grâce à quoi des complexes RNP contenant les segments d'ARNv génomiques dudit virus sont formés et lesdites particules virales infectieuses sont produites par lesdites cellules en l'absence d'un virus assistant et où lesdites cellules ne produisent pas d'interféron.

2. Procédé tel que revendiqué dans la revendication 1 dans lequel un ou plusieurs vecteurs d'expression supplémentaires sont utilisés dans lesdites cellules pour exprimer une ou plusieurs protéines sélectionnées de ladite nucléoprotéine et des sous-unités de ladite ARN polymérase dépendante de l'ARN.

3. Procédé tel que revendiqué dans la revendication 1 ou 2 dans lequel une lignée cellulaire qui est capable d'exprimer une ou plusieurs de ladite nucléoprotéine et des sous-unités de ladite ARN polymérase dépendante de l'ARN est utilisée.

4. Procédé tel que revendiqué dans l'une quelconque des revendications 1 à 3 dans lequel ledit virus est un virus réassorti ayant des segments d'ARNv dérivés de plus d'un virus parent.

5. Procédé tel que revendiqué dans l'une quelconque des revendications 1 à 4 dans lequel lesdites cellules sont des cellules Vero.

6. Procédé tel que revendiqué dans l'une quelconque des revendications 1 à 5 dans lequel lesdits vecteurs d'expression définis dans la revendication 1 expriment des segments d'ARNv génomiques dudit virus.

7. Procédé tel que revendiqué dans l'une quelconque des revendications 1 à 6 comprend en outre l'amplification desdites particules virales formées par une ou plusieurs étapes d'infections cellulaires subséquentes en utilisant le même type de cellules ou un type de cellules différent.

8. Procédé tel que revendiqué dans l'une quelconque des revendications 1 à 7 qui comprend en outre l'isolation de particules virales infectieuses.

9. Procédé tel que revendiqué dans l'une quelconque des revendications 1 à 8 qui comprend en outre une étape d'élimination ou d'atténuation virales.

10. Procédé tel que revendiqué dans l'une quelconque des revendications 1 à 9 dans lequel tous les vecteurs d'expression requis sont co-transfectés dans lesdites cellules en utilisant un réactif de transfection liposomale, une électroporation ou une précipitation de phosphate de calcium.

11. Procédé tel que revendiqué dans l'une quelconque des revendications 1 à 10 dans lequel lesdits vecteurs d'expression sont tous des plasmides.

12. Procédé tel que revendiqué dans l'une quelconque des revendications 1 à 11 dans lequel lesdits vecteurs d'expression définis dans la revendication 1 se composent de vecteurs d'expression séparés pour l'expression de chaque segment d'ARNv dudit virus.

13. Procédé tel que revendiqué dans l'une quelconque des revendications 1 à 12 dans lequel l'expression de chaque segment d'ARNv est sous le contrôle d'une séquence promotrice dérivée d'un promoteur de Poll d'un mammifère.

14. Procédé tel que revendiqué dans la revendication 13 dans lequel ladite séquence promotrice est une séquence de promoteur de Poll humain tronquée constituée de nucléotides -250 à -1 du promoteur natif correspondant ou d'un dérivé fonctionnel de celui-ci.

15. Procédé tel que revendiqué dans l'une quelconque des revendications 1 à 14 dans lequel la séquence de codage pour chaque segment d'ARNv dans lesdits vecteurs d'expression est suivie par une séquence de ribozyme ou un terminateur de transcription qui produit une extrémité 3' correcte de chaque dit ARN.

16. Procédé tel que revendiqué dans la revendication 2 dans lequel l'expression d'une ou de plusieurs protéines virales desdits vecteurs d'expression supplémentaires est sous le contrôle d'une séquence régulatrice sélectionnée du promoteur tardif majeur de l'adénovirus de type 2 lié à la séquence de tête tripartite épissée de l'adénovirus humain de type 2 ou du promoteur précoce-immédiat du cytomégalovirus humain, ou d'un dérivé fonctionnel de ladite séquence régulatrice.

17. Procédé tel que revendiqué dans l'une quelconque des revendications 1 à 16 qui comprend en outre l'incorporation d'un virus tué ou atténué dans une composition de vaccin.

18. Procédé tel que revendiqué dans l'une quelconque des revendications 1 à 17 dans lequel ledit virus a au moins un segment d'ARNv capable de diriger l'expression d'une séquence hétérologue audit virus dans des cellules cibles infectées par ledit virus.

19. Procédé tel que revendiqué dans la revendication 18 dans lequel ladite séquence hétérologue audit virus code un peptide antigénique ou un polypeptide antigénique et qui comprend en outre l'incorporation dudit virus dans une composition de vaccin.

20. Cellules modifiées telles que produites dans la revendication 1 ou 5.

21. Procédé pour produire des particules virales infectieuses d'un virus à polarité négative segmenté qui comprend la culture des cellules modifiées telles que revendiquées dans la revendication 20.

22. Procédé pour générer dans des cellules cultivées des particules virales infectieuses du virus de la grippe A, ledit procédé comprenant le fiait :
de fournir une première population de cellules qui sont capables de soutenir la croissance dudit virus et qui ont été modifiées afin de fournir (a) les ARNv génomiques dudit virus et (b) une nucléoprotéine et une ARN polymérase dépendante de l'ARN grâce à quoi des complexes RNP contenant lesdites ARNv génomiques sont formés et lesdites particules virales infectieuses sont assemblées, lesdites ARNv génomiques sont directement exprimées dans lesdites cellules sous le contrôle d'un promoteur de Poll d'un mammifère ou d'un dérivé fonctionnel de celui-ci en l'absence d'un virus assistant, et où lesdites cellules de produisent pas d'interféron.

23. Procédé de la revendication 22 comprenant en outre l'amplification desdites particules virales assemblées par une ou plusieurs étapes d'infections cellulaires subséquentes utilisant le même type de cellules ou un type de cellules différent.

24. Procédé tel que revendiqué dans la revendication 22 ou 23 qui comprend en outre l'isolation des particules virales infectieuses.

25. Procédé tel que revendiqué dans l'une quelconque des revendications 22 à 24, qui comprend en outre une étape d'élimination ou d'atténuation virale.

26. Procédé tel que revendiqué dans l'une quelconque des revendications 22 à 25 qui comprend en outre l'incorporation de particules virales tuées ou atténuées dans une composition de vaccin.

27. Procédé tel que revendiqué dans l'une quelconque des revendications 22 à 25 dans lequel ledit virus a un segment d'ARNv au moins capable de diriger l'expression d'une séquence hétérologue audit virus dans des cellules cibles infectées par ledit virus et qui comprend en outre l'incorporation dudit virus, si cela est approprié après atténuation ou élimination, dans une composition pharmaceutique conjointement avec un diluent ou un porteur pharmaceutiquement acceptable.

28. Procédé tel que revendiqué dans l'une quelconque des revendications 22 à 27, dans lequel lesdites cellules sont des cellules Vero.

29. Cellules modifiées telles que produites dans la revendication 22 ou 28.

30. Procédé pour produire des particules virales infectieuses d'un virus à polarité négative segmenté qui comprend la culture de cellules modifiées telles que revendiquées dans la revendication 29.

31. Procédé de l'une quelconque des revendications 7 à 15 et 17 à 19 dans lequel les cellules utilisées pour amplification sont des cellules MDBK ou des cellules MDCK.

32. Procédé de l'une quelconque des revendications 23 à 28, dans lequel les cellules utilisées pour amplification sont des cellules MDBK ou des cellules MDCK.

33. Procédé des revendications 1 à 19, 21 à 28 ou 30 à 32, dans lequel les cellules sont des cellules EcR-293NP.
